# EUROPÄISCHE PATENTSCHRIFT

(11) **EP 1 916 965 B1**
(45) Veröffentlichungstag und Bekanntmachung des Hinweises auf die Patenterteilung: **25.02.2015**
(21) Anmeldenummer: 06818238.5
(22) Anmeldetag: 19.07.2006
(51) Int. Cl.: A61F 2/07

(54) **STENTGRAFT-PROTHESE**
STENT-GRAFT PROSTHESIS
GREFFE D'ENDOPROTHESE

(30) Priorität: 19.07.2005 DE 102005034617; 27.04.2006 DE 102006020687
(43) Veröffentlichungstag der Anmeldung: 07.05.2008
(73) Patentinhaber: Aesculap AG, 78532 Tuttlingen (DE); TuTech Innovation GmbH, 21079 Hamburg (DE); Technische Universität Hamburg-Harburg, 21071 Hamburg (DE)
(72) Erfinder: GOLDMANN, Helmut, 78532 Tuttlingen (DE); IMIG, Herbert, 14109 Berlin (DE); MORLOCK, Michael, 20359 Hamburg (DE); GEBERT, Anne, 20146 Hamburg (DE)
(74) Vertreter: Patentanwälte Ruff, Wilhelm, Beier, Dauster & Partner
(86) Internationale Anmeldenummer: PCT/EP2006/007097
(87) Internationale Veröffentlichungsnummer: WO 2007/028452

(56) Entgegenhaltungen:
- US-A- 5 693 088
- US-B1- 6 729 356

## Beschreibung

Die Erfindung betrifft eine Stentgraft-Prothese mit einer toroidförmig aufweitbaren mindestens zwei Öffnungsansätze aufweisenden Hülle aus flexiblem Material zur inneren Auskleidung eines arteriellen Aneurysmas und einem der Hülle zugeordneten Aorteneinsatz in Form einer Gefäßprothese mit aufweitbaren Stützstrukturen zur Befestigung des Aorteneinsatzes in der Hülle im Bereich deren Öffnungsansätze.

Ein Stentgraft ist ein dauerhaft im Körper verbleibendes Implantat, das endovaskulär, d.h. mittels Katheter zur Behandlung von Bauchaortenaneurysmen (AAA) oder Aneurysmen der thorakalen Aorta (TAA) platziert wird. Durch die Implantation eines Stentgrafts soll eine dauerhafte Verankerung des Implantates im Blutgefäß ermöglicht werden, wodurch die Ausschaltung des Aneurysmas vom Blutstrom gewährleistet wird.

Bekannt sind Stentgrafts, die aus unterschiedlichen Graft-Materialien bestehen, an deren Außen- oder Innenseite oder auch eingebunden zwischen zwei Materialschichten, unterschiedlich ausgebildete metallische Stentstrukturen angebracht sind. Proximal und/oder distal befindet sich häufig ein ungecoverter Stent und/oder Haken, die eine Verankerung ermöglichen. Es sei auf folgende Druckschriften hingewiesen: US 6,517,570 B1, WO 04/037116, WO 01/49211, WO 01/21102 A1, EP 1 086 663 A1, US 6,203,568 B1, WO 00/33769 A1, EP 0 947 179 A2, US 5,938,696, WO 99/29262 A1, WO 98/44870 A1, WO 98/53761 A1, EP 0 893 108 A2, US 6,361,556 B1.

Neuere Patentanmeldungen beschäftigen sich mit Konzepten, welche die bekannten Probleme der Stentgrafts vermeiden sollen. Es werden Taschen an den Stentgrafts angebracht, die mit inkompressiblen Agenzien gefüllt die Expansion der Hülle des Stentgrafts bewirken sollen (vgl. US 2003/0074058 A1 und WO 03/032869 A1). Bekannt sind auch inflatierbare Manschetten bzw. flüssigkeitsdichte Kammern, die durch Füllen mit einer Flüssigkeit die Expansion des Stentgrafts bewirken (US 2002/0116048 A1, WO 99/39662, WO 03/053288 A1). Die US 2003/0074048 A1 beschreibt einen Stentgraft mit einer inneren dichten Hülle und einer äußeren durchlässigen Hülle mit dem Ziel, über die durchlässige Hülle Serum in den Aneurysmensack abzuscheiden.

Ein weiteres Konzept beinhaltet einen Stentgraft mit einem Port in der Wand, durch den embolisierend wirkende Materialien in das Aneurysma eingebracht werden können (US 2002/0169497 A1 und US 2003/0014075 A1). Aus der US 6,729,356 B1 ist eine toroide, an der Prothese fixierte Membran bekannt, welche mit Blut oder anderen Substanzen ausgefüllt werden kann. Eine Abdichtung im proximalen und distalen Bereich, welche separat von der Prothese eingebracht werden kann, ist aus der US 6,729,356 B1 bekannt. Dort wird die Prothese mit Zacken oder Federhaken mit der Membran verbunden. Füllmaterial kann in den Prothesensack eingefüllt werden und härtet dann aus. Es bildet eine Barriere gegen den Druck und eine Fixierung der Prothese. Die WO 04/037116 beschreibt eine doppelwandige Prothese, die eine äußere Membran besitzt, die elastisch expandiert wird. Die Füllung zwischen Stentgraft und äußerer, dichter Membran erfolgt mit Blut oder anderen Füllstoffen, die mittels eines Katheters appliziert werden können. Eine ähnliche Möglichkeit sieht die US 2004/0098096 A1 vor. Dort besteht die Hülle aus elastischem Material, das sich unter dem Druck der einströmenden Flüssigkeit, z.B. Blut, elastisch aufweitet und an die Innenseite des Aneurysmas anlegt. Dort ist auch die Möglichkeit der Ausbildung von Bifurkationen beschrieben. Die WO 2004/004603 A1 sieht vor, bei einem abdominalen Aortenaneurysma (AAA) zwei Aorteneinsätze vorzusehen, die im Aneurysma parallel verlaufen und sich dann an der Bifurkation in die Beinarterien aufteilen. Aus der US 5,693,088 bekannte Gefäßprothesen umfassen einen biokompatiblen Gefäßeinsatz, ein radial aufweitbares Gerüst, welches den Gefäßeinsatz umgibt sowie punktionsfreie Befestigungsvorrichtungen zur Fixierung der Prothese innerhalb des Gefäßes. Die Befestigungsvorrichtungen können die Form eines toroidalen Umhüllungselementes aufweisen, welches aus einem den Gewebeeinwuchs fördernden Material besteht. Das Umhüllungselement weitet sich nach dem Einsetzen der Prothese unter dem Druck einströmender Flüssigkeit, beispielsweise Blut, auf und legt sich an die Innenseite der Gefäßwand an.

Der Erfindung liegt die Aufgabe zugrunde, die Schwachstellen konventioneller Stentgrafts zu minimieren. Endolekagen, insbesondere durch retrograden Kollateralarterienfluss, Migration des Stentgrafts sollen vermieden werden.

Die Erfindung ist dadurch gekennzeichnet, dass die Hülle als faltbares Toroid ausgebildet ist und der Form eines Aneurysmas angepasst ist. Vorzugsweise besitzt die Hülle eine nicht elastische druckunabhängige Volumenausdehnbarkeit, die mindestens dem Volumen des Aneurysmas entspricht. Zumindest im Mittelbereich des Toroids ist die Hülle aus einem im wesentlichen nicht elastisch dehnbaren Material gebildet. Die Hülle vermag im gefüllten Zustand eine der Innenform des Aneurysmas entsprechende Form anzunehmen. Die Hülle kann sich aufgrund des arteriellen Blutdrucks ohne Ausbildung eines Gegendrucks entfalten. Da die Form der Hülle der eines Aneurysmas nachgebildet ist, kann sich die Hülle an allen Stellen des Aneurysmas an dessen Innenwand anlegen. Dadurch wird einerseits ein sicherer Halt der Hülle im Aneurysma erhalten, andererseits werden Kollateralarterien sicher verschlossen, wodurch Endolekagen, insbesondere ein retrograder Kollaterialarterienfluss, vermieden werden können. Dadurch, dass die Hülle bereits die Form eines Aneurysmas besitzt und drucklos entfaltbar ist, kommt der volle arterielle Druck als Anpressdruck zur Anwendung. Es ist also kein durch die Hüllenwandung erzeugter Gegendruck zu überwinden, wie dies bei elastisch unter Druck aufweitbaren Hüllen der Fall ist.

Der Hülle ist ein Aorteneinsatz zugeordnet, der in die Hülle einsetzbar ist. Die Hülle ist aber auch für sich alleine, d.h. ohne Aorteneinsatz, einsetzbar. Insbesondere ist eine gesonderte Implantation angezeigt, wenn eine rasche Blutdruckstabilisierung bei bereits perforiertem Aneurysma im Vordergrund steht. Die Öffnungsansätze, die den proximalen und distalen Enden des Aneurysmas entsprechen, sind gegenüber dem Lumen des Toroids verjüngt und dem Innendurchmesser der Aorta in diesen Bereichen angepasst. Die erfindungsgemäße Stentgraft-Prothese, insbesondere die Hülle, kann in verschiedenen Größen und Formen vorrätig gehalten werden, um den jeweils vorliegenden Größenverhältnissen Rechnung zu tragen. Die Implantation der Hülle ohne Aorteneinsatz ist dort angezeigt, wo es in erster Linie auf eine Abdichtung der Ansätze der Kollateralen ankommt, um einen Rückfluß von Blut aus den Kollateralen in das Aneurysma zu verhindern. Das Volumen des Aneurysmas innerhalb der Hülle bleibt dann vom Blut durchströmt.

Wie bereits erwähnt, besitzt die Hülle eine drucklose Volumenausdehnbarkeit, die mindestens dem Volumen des Aneurysmas entspricht, wobei die Hülle im Vergleich zur Größe des Aneurysmas vorzugsweise überdimensioniert ist. Durch eine solche Überfütterung der Hülle im Aneurysma kann die Hülle beim Anlegen an die Innenwand des Aneurysmas Falten aufweisen. Dies stört jedoch nicht. In der Praxis kann die Hülle eine bis ca. 5-fache, insbesondere 1,1- bis 2-fache Größe eines Aneurysmas aufweisen.

Mit Vorteil wird die Hülle von einem mindestens zum Teil faltbaren Material gebildet. Da sie in der Regel mit Hilfe eines Katheters intraarteriell bis zum Aneurysma gebracht und dort platziert wird, kann sie in einfacher Weise auf das Innenlumen des Katheters zusammengefaltet werden und danach entfaltet. Zumindest am Übergang des Körpers des Toroids bis zu den Öffnungsansätzen ist bei einer bevorzugten Ausführungsform das Hüllenmaterial elastisch dehnbar ausgebildet. Dadurch kann die Hülle im Bereich der Öffnungsansätze faltenfrei an die Innenseite der Aorta angelegt werden, wodurch die abdichtende Befestigung an der Aorteninnenwand, insbesondere mit Hilfe von Stents, erleichtert wird. Der Übergang vom elastischen Material im Bereich der Öffnungsansätze zu einem nicht elastischen Material im Bereich des Volumens des Toroids kann abrupt sein, indem das nicht elastische Material am elastischen Material befestigt und im Bereich der Befestigung vorzugsweise gefältelt ist, um eine Ausdehnung zu ermöglichen. Es sind aber auch kontinuierliche Übergänge möglich, beispielsweise indem sich in Umfangsrichtung dieser Bereiche Längsabschnitte aus nicht elastischem Material mit Längsabschnitten aus elastischem Material abwechseln und allmähliche Übergänge in der Breite besitzen. Als elastisches Material eignet sich bioverträglicher Kautschuk, wie Silikonkautschuk oder auch elastisches Polyurethan. Bei elastisch dehnbarem Material können unterschiedlich dehnbare Wandungsabschnitte auch durch verschiedene Wandstärken erhalten werden. Eine toroidförmige Hülle aus elastischem Material kann durch Beschichten eines aufblasbaren toroidförmigen Kerns, zum Beispiel eines Ballons, mit einer Lösung eines elastischen Kunststoffs, zum Beispiel Polyurethan, hergestellt werden.

In einer weiteren Ausführungsform weist die Hülle eine rauhe Innenoberfläche auf. Die Innenoberfläche kann insbesondere mechanisch aufgerauht sein. Durch die Rauhigkeit an der Hülleninnenseite wird mit besonderem Vorteil ein Verkleben der Hüllenwandung vermieden und insgesamt die Handhabung der Hülle verbessert.

Die Hülle kann weiterhin eine glatte Aussenoberfläche aufweisen. Erfindungsgemäß ist es insbesondere vorgesehen, dass die Hülle an ihrer Aussenoberfläche mit einem Gleitmittel, insbesondere in Form einer Beschichtung, versehen ist. Bei dem Gleitmittel kann es sich beispielsweise um Glycerin handeln.

Bei einer bevorzugten Ausführungsform der Erfindung ist die Hülle auf ihrer Außenseite mit einem Kleber zur Haftung und permanenter Abdichtung an der Aneurysmeninnenwand versehen. Normalerweise verankert sich eine Stentgraft-Prothese mehr oder weniger mechanisch in einem Aneurysma durch ihre ballonförmige bzw. toroidförmige Gestalt. Eine Verklebung zwischen Hülle und Aneurysmeninnenwand führt zur einer aktiven Fixierung und Abdichtung. Als Kleber eignen sich vorzugsweise biologische Kleber, insbesondere solche, die im Zusammenhang mit der Blutgerinnung stehen. Geeignete Kleber sind beispielsweise Fibrin- und Thrombinkleber. Die Aktivität des Klebers kann zeitlich gesteuert sein, so dass die Klebewirkung erst nach einer vorbestimmten Zeit eintritt. So kann die Klebeschicht mit einer biologisch abbaubaren Schutzschicht maskiert sein, wodurch verhindert werden kann, dass die Klebekraft vorzeitig aktiv ist. Zur Maskierung kann ein Gel vorgesehen sein, das gleichzeitig als anfängliches Gleitmittel wirkt und die Entfaltung der Hülle begünstigt. Bei Kombinationen von Hülle und Aorteneinsatz ist es weiterhin vorteilhaft, wenn die Innenseite der Hülle und/oder die Außenseite des Aorteneinsatzes die Blutkoagulation fördernde Mittel aufweisen. Solche Mittel können wiederum mit Vorteil als Beschichtung vorliegen. In den Zwischenraum zwischen Hülle und Aorteneinsatz einströmendes Blut verfestigt sich durch die Blutgerinnung sehr rasch uns sorgt für stabile Verhältnisse.

Mit Vorteil ist die Hülle mindestens zum Zeitpunkt der Implantation, vorzugsweise bleibend, flüssigkeitsdicht ausgebildet. Dies ist üblich und für eine gute Funktion auch zweckmäßig. Geeignete Materialien für die Hülle sind Silikonkautschuk, Polyurethan, Polyethylen und Polytetrafluorethylen. In einer bevorzugten Ausführungsform besteht die Hülle aus Polyurethan. Bei dem Polyurethan handelt es sich insbesondere um ein aliphatisches Polyurethan, welches vorzugsweise linear ist. Die Hülle hat normalerweise eine Wandstärke im Bereich von 0,05 bis 2 mm. Für besondere Fälle ist es möglich, das Material der Hülle so zu gestalten, dass es ähnlich wie bei einer Gefäßprothese nur anfänglich flüssigkeitsdicht ist. So kann die Hülle auch aus textilem Material, insbesondere einem Gewirk oder Gewebe ausgebildet sein, das anfänglich mit einem resorbierbaren Imprägnierungsmittel abgedichtet ist. Es kann sogar vorgesehen sein, der Hülle eine mikrofeine netzartige Stützstruktur aus nicht resorbierbarem Material zu verleihen, die mit resorbierbarem Material abdichtend beschichtet ist. Eine solche Netzstruktur kann ebenfalls textiler Art sein. Textile oder sonstige poröse Strukturen ermöglichen das Durchwachsen mit biologischem Gewebe und damit eine zusätzliche Verankerung.

Wie oben bereits erwähnt, können die Hülle und der ihr zugeordnete Aorteneinsatz separat vorliegen. Diese Ausführungsform ermöglicht es, die beiden Teile der Stentgraft-Prothese zeitlich nacheinander zu implantieren, d.h. zuerst die Hülle und danach den Aorteneinsatz. Bei dieser Ausführungsform sind der Hülle bzw. ihren Öffnungsansätzen eigenständige Befestigungsmittel zugeordnet, z.B. Stents, insbesondere solche, die zusätzliche Verankerungselemente zur Verankerung an der Aorteninnenwand aufweisen. Da die Hülle dann bereits mit Blut befüllt ist und sich an der Innenwand des Aneurysma angelegt hat, kann der nachfolgend zu implantierende Aorteneinsatz einen Mantel besitzen, der blutdicht ist (zum Beispiel aus non-woven Polytetrafluorethylen), da es dann nicht mehr erforderlich ist, dass Blut durch die Wandung bzw. den Mantel des Aorteneinsatzes in den Zwischenraum zwischen Hülle und Aorteneinsatz nachströmt. Bei dieser Ausführungsform ist der Aorteneinsatz an seinen freien Enden mit den Öffnungsansätzen der Hülle abdichtend verbindbar. Dies kann dadurch geschehen, dass die freien Enden des Aorteneinsatzes flächig und dicht an die Innenwand der Hülle im Bereich der Öffnungsansätze gedrückt werden. Es können auch zusätzliche Dichtungs- bzw. Klebemittel vorgesehen sein. Geeignet sind beispielsweise Schäume oder Vliese aus Kollagen oder Polyurethan.

In bekannter Weise kann die Stentgraft-Prothese auch für die Implantation in einem Aneurysma ausgebildet sein, an dem die Arterie eine Bifurkation aufweist, wie dies bei Bauchaortenaneurysmen (AAA) häufig der Fall ist. Entsprechend weisen dann die Hülle und gegebenenfalls der Aorteneinsatz zwei distale Öffnungsansätze bzw. Öffnungen auf.

Für die meisten Anwendungsfälle reicht es aus, wenn der Aorteneinsatz an seinen freien Enden mit den Öffnungsansätzen der Hülle bereits abdichtend verbunden ist, bevor die Stentgraft-Prothese implantiert wird. Zwischen Hülle und Aorteneinsatz ist dann eine Kammer ausgebildet, die nach der Implantation vergrößerbar ist. Bei dieser Ausführungsform ist die Wandung bzw. der Mantel des Aorteneinsatzes mindestens anfänglich für Blut durchlässig, so dass die Kammer während oder nach der Implantation durch hindurchströmendes Blut unter dem arteriellen Blutdruck auffüllbar ist und sich die Hülle an die Innenwandung des Aneurysmas anlegt. Wenn die Kammer gefüllt ist und das Blut koaguliert bzw. gerinnt, dichtet sich der Aorteneinsatz automatisch ab. Als Aorteneinsatz kann beispielsweise eine handelsübliche Gefäßprothese verwendet werden. Diese kann außerhalb der endständigen Befestigungsbereiche auch plissiert sein. Bei dieser Ausführungsform kann die Kammer zwischen Hülle und Aorteneinsatz ihrerseits Mittel enthalten, die die Blutkoagulation begünstigen. Dies kann wiederum in Form von Beschichtungen vorgesehen sein oder in Form von pulverförmigen oder flüssigen Zusätzen.

Sind Aorteneinsatz und Hülle bereits miteinander verbunden, so kann die Befestigung der Stentgraft-Prothese im Bereich des Aneurysma in an sich bekannter Weise erfolgen, beispielsweise durch proximale und/oder distale Stents. Diese können selbstaufweitend oder beispielsweise mit einem Ballon aufweitbar sein. Die Hülle selbst kann an ihrer Außenseite Befestigungsmittel aufweisen, mit denen sie sich an der Aorteninnenwand fixiert. Diese Befestigungsmittel können in Form von Krallen oder Haken vorliegen, die auch durch die Hüllenwandung von innen nach außen durchgreifen können.

Liegen Hülle und Aorteneinsatz getrennt vor, dann sind zusätzliche Befestigungsmittel zur Befestigung des Aorteneinsatzes an der Innenseite der Öffnungsansätze der Hülle vorgesehen. Diese können wiederum in Form von Krallen oder Haken ausgebildet sein. Die Stents können zusätzlich zu ihrer Expansionsfähigkeit noch solche Befestigungsmittel aufweisen, die durch die Wandung des Aorteneinsatzes hindurch bis in die Hülle greifen. Bei einer bevorzugten Ausführungsform weisen die Innenseite der Hülle und die Außenseite des Aorteneinsatzes im Befestigungsbereich Elemente eines Klettenverschlusses (Haken und Ösen) auf, die sich gegenseitig nach der Implantation ineinander verkrallen. Weiterhin ist es möglich, die Berührungsflächen zwischen Hülle und Aorteneinsatz rau zu gestalten und/oder mit klebenden Verbindungsmitteln auszurüsten.

Weitere Merkmale der Erfindung ergeben sich aus der nachfolgenden Beschreibung von bevorzugten Ausführungsformen in Verbindung mit der Zeichnung und den Unteransprüchen. Hierbei können die einzelnen Merkmale jeweils für sich alleine oder in Kombination miteinander verwirklicht sein.

### Figurenbeschreibung

In der Zeichnung zeigen
- Figur 1:: ein Bauchaortenaneurysma, in das eine Hülle gemäß der Erfindung eingesetzt ist,
- Figur 2:: die Ausführungsform nach Figur 1, wobei die Hülle mit Stents befestigt ist,
- Figur 3:: ein Bauchaortenaneurysma mit eingesetzter Hülle, die am proximalen Ende Befestigungsmittel zur Befestigung eines Aorteneinsatzes aufweist,
- Figuren 4a, b, c und d:: verschiedene Befestigungselemente,
- Figur 5a und b:: Befestigungselemente gemäß einer anderen Ausführungsform,
- Figur 6:: ein Bauchaortenaneurysma mit einer erfindungsgemäßen Kombination aus Hülle und Aorteneinsatz,
- Figur 7:: eine zusammengefaltete und in einem Katheter angeordnete Hülle im Zustand zur Einführung in ein Aneurysma.

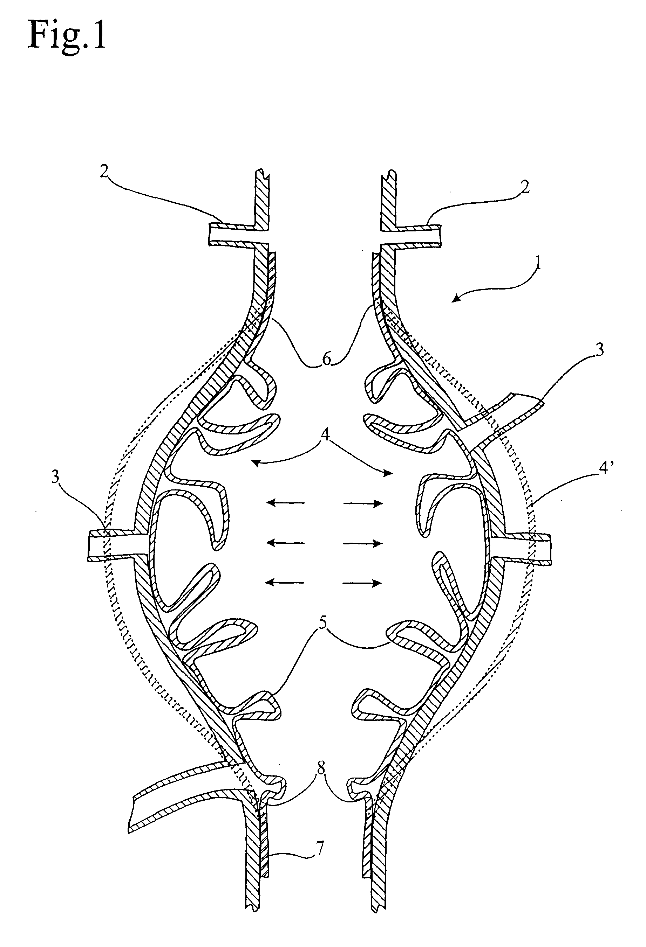

Die verschiedenen Ausführungsformen sind in der Zeichnung in schematischer Darstellung gezeigt. Das in Figur 1 dargestellte Bauchaortenaneurysma (AAA) befindet sich unterhalb der Abgänge der Nierenarterien. Die zu den beiden Nieren abzweigenden Arterien 2 sind vom Aneurysma nicht betroffen und müssen bei der Platzierung einer Stentgraft-Prothese offen bleiben. Dagegen können Kollateralarterienäste 3, die im Bereich des Aneurysmas abzweigen, verschlossen werden. In das Bauchaortenaneurysma 1 eingelegt ist eine toroidförmig ausgebildete zusammenfaltbare Hülle 4, die aus Polyurethan besteht. Die Hülle hat eine Wandstärke von ca. 200 µm. Die Hülle erstreckt sich über die gesamte Länge des Aneurysmas und reicht bis in gesunde proximale und distale Abschnitte der Baucharterie. Die Hülle 4 besitzt im drucklos gefüllten Zustand eine toroide Form und besitzt eine deutliche Überfütterung, d.h. Überdimensionierung, unter Bildung von Falten 5 innerhalb des Aneurysmas (Die Falten 5 sind in Figur 1 übertrieben groß dargestellt). Das heißt in diesem Bereich könnte die Hülle auch ein größeres oder sich vergrößerndes Aneurysma vollständig ausfüllen. Die volle Größe der Hülle 4' ist in Figur 1 gestrichelt dargestellt. Im proximalen und distalen Bereich besitzt die Hülle Öffnungsansätze 6 und 7, die gegenüber dem Mittelbereich der Hülle deutlich verjüngt sind und nur eine leicht konische Form besitzen. Im Durchmesser sind die Öffnungsansätze 6 und 7 dem Innendurchmesser der Baucharterie angepasst. Im Bereich der Öffnungsansätze 6 und 7 besteht die Hülle aus elastisch dehnbarem Material, das ab einer angedeuteten Grenze 8 in ein flexibles nicht elastisches Material übergeht. Der Übergang kann allmählich ausgebildet sein. Während somit die Hülle im Bereich der Öffnungsansätze elastisch aufweitbar ist und mit geeigneten Befestigungselementen an der Innenwand der Arterie abdichtend angelegt werden kann, braucht der das eigentliche Volumen der Hülle ausmachende Mittelabschnitt nicht elastisch dehnbar ausgebildet zu sein und wird aufgrund seiner Überdimensionierung unter Einwirkung des arteriellen Blutdrucks (vgl. Pfeile) unter Faltenbildung 5 an die Aneurysmenwand gedrückt.

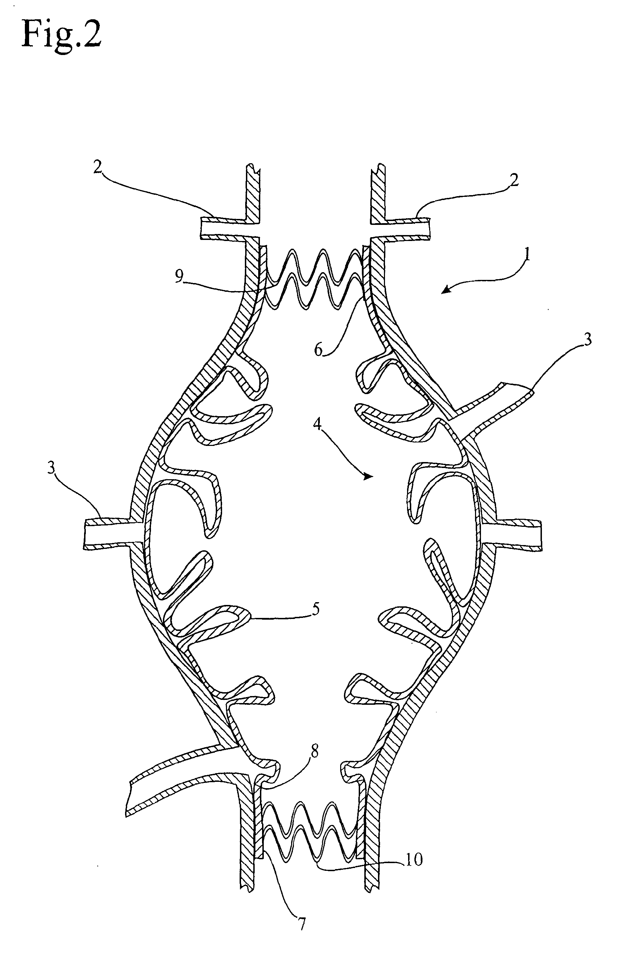

Figur 2 zeigt die gleiche Ausführungsform, bei der als Befestigungsmittel am proximalen und am distalen Öffnungsansatz 6 und 7 der Hülle ein proximaler und distaler Stent 9 und 10 eingesetzt ist, der entweder radial nach außen federnd ausgebildet oder durch mechanische Mittel aufgeweitet ist, um die Hülle an die Arterieninnenwand zu drücken und dort zu fixieren. Die Stents haben eine axiale Länge, die in etwa in der Größe der axialen Länge der Öffnungsansätze 6 und 7 liegen.

Die Hülle nach Figur 2 kann in dem in Figur 2 dargestellten fixierten Zustand verbleiben. Zusätzlich kann eine nachfolgende Implantation eines Aorteneinsatzes vorgesehen sein.

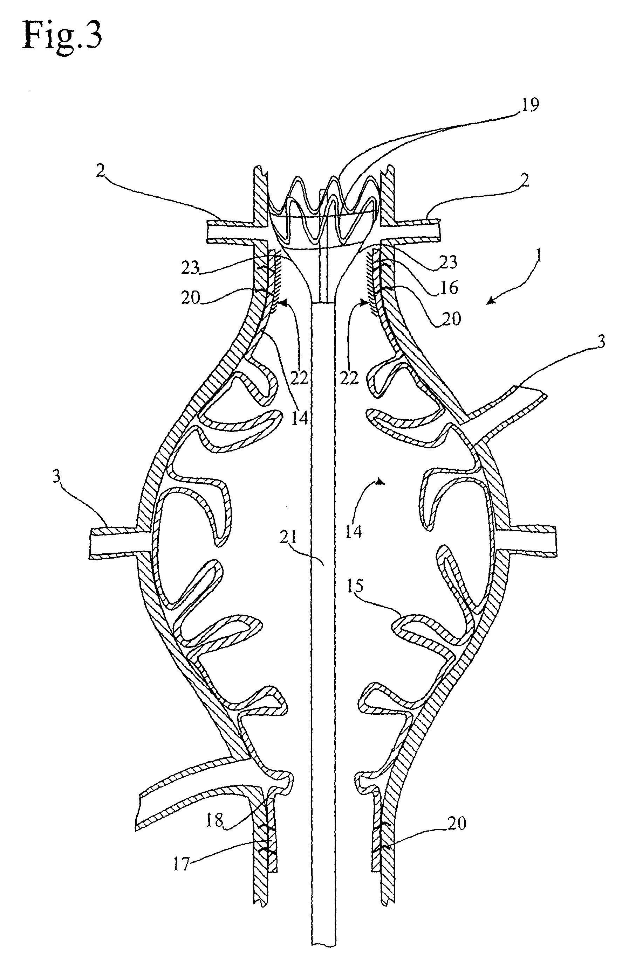

Figur 3 zeigt wiederum bei einem Bauchaortenaneurysma die Vorbereitung zur Fixierung eines Aorteneinsatzes. Eine Hülle 14 ist im wesentlichen gleich ausgebildet wie bei den Ausführungsformen nach den Figuren 1 und 2. Entsprechende Teile haben um die Zahl 10 erhöhte Bezugszahlen. Die Hülle 14 ist an ihren proximalen und distalen Öffnungsabschnitten 16 und 17 mit Hilfe von Häkchen 20, die in das Hüllenmaterial eingebettet sind, an der Aortenwand verkrallt. Zusätzlich ist im proximalen Bereich ein Stent 19 vorgesehen, der mit dem oberen Ende eines Aorteneinsatzes 23 verbunden ist und dieses nach Platzierung des Aorteneinsatzes zusammen mit der Hülle gegen die Aorteninnenwandung drückt. Der Aorteneinsatz ist in Figur 3 in einem Zustand gezeigt, in dem er zum größten Teil noch in einem rohrförmigen Platzierungsinstrument 21 angeordnet ist.

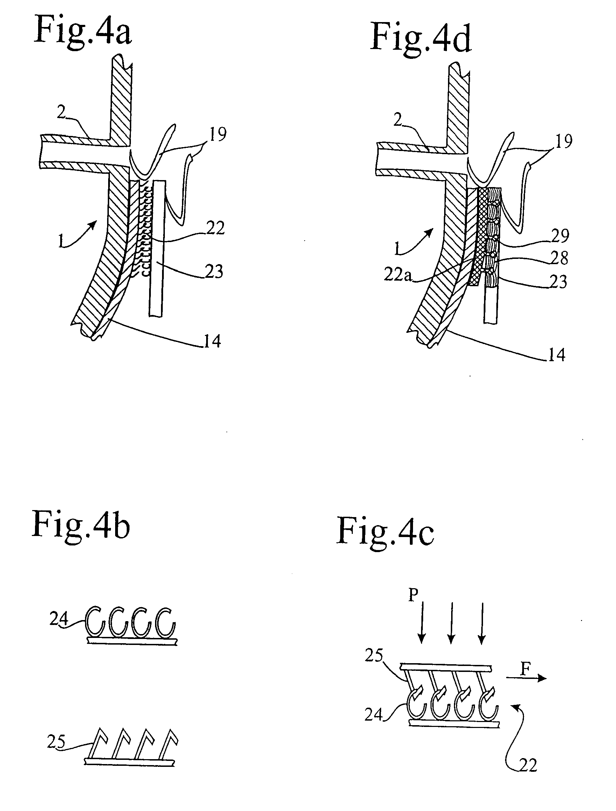

An der Innenseite des proximalen Öffnungsabschnittes 16 der Hülle 14 befinden sich flächig angeordnete Elemente eines Klettverschlusses 22, deren Gegenstücke auf der Außenseite des proximalen Endes des Aorteneinsatzes 23 angeordnet sind, wie dies in Figur 4a angedeutet ist. Die Elemente des Klettverschlusses 22 bestehen aus Ösen 24 und Haken 25, wie sie in vergrößertem Maßstab in Figur 4b dargestellt sind. Figur 4c zeigt das Ineinandergreifen der Elemente 24 und 25 unter Bildung des Klettverschlusses 22. Beim Einsetzen eines entsprechend ausgebildeten an seiner Außenwand Klettverschlusselemente aufweisenden Aorteneinsatzes wird dieser an seinem proximalen Ende beispielsweise durch Ballondilatation gegen die Innenwand des Öffnungsabschnittes 16 gedrückt, wodurch der Klettverschluss 22 bleibend verschlossen wird und gegebenenfalls durch einen Stent 19 gesichert wird. Im distalen Bereich kann ebenfalls ein entsprechender Klettverschluss vorgesehen sein oder auch lediglich ein Stent. Es ist auch möglich, die textile Wandung 28 des Aorteneinsatzes 23 als Teil, nämlich als Ösen- bzw. Flanschteil, eines Klettverschlusses 22 vorzusehen. Die Innenwand des Öffnungsabschnittes 16 weist dann die Haken bzw. Knopfteile 29 des Klettverschlusses auf, wie dies in Figur 4d schematisch dargestellt ist. Der Klettverschluss 22 bzw. der Haken bzw. Knopfteil 22a kann umlaufend gerade, zick-zack-förmig in Form von parallelen Streifen, die versetzt angeordnet sind, ausgebildet sein.

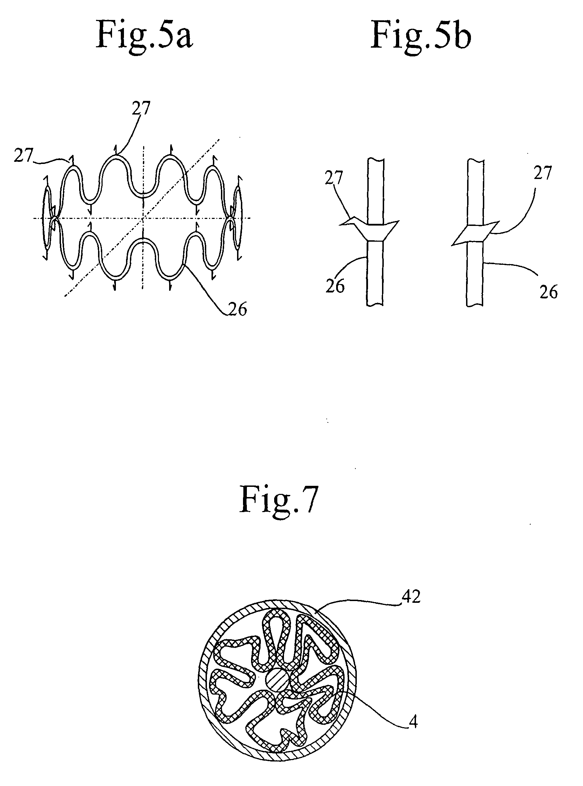

Figur 5a zeigt einen wellenförmig oder mäanderförmig gebogenen Stent 26, der beispielsweise aus selbstauffederndem Draht gebildet ist. Im Vergleich zu einem normalen Stent dieser Art besitzt der Stent nach Figur 5a an den Wellentälern und Wellenbergen radial nach außen gerichtete Vorsprünge 27, mit deren Hilfe der Stent sich in einer Wandung, zum Beispiel des Aorteneinsatzes, verkrallen kann. Die Verkrallung kann sogar so weit gehen, dass die Vorsprünge beispielsweise durch die Wandung hindurchragen bis in das Material der dahinterliegenden Hülle. Ein solcher Stent kann auch zur Befestigung der Hülle in der Aortenwand verwendet werden. Figur 5b zeigt Beispiele für solche Vorsprünge. Diese können, wenn der Stent beispielsweise zwischen Hülle und Aorteneinsatz angeordnet ist, auch beidseitig überstehen, wie dies in Figur 5b gezeigt ist. Die Stents und sonstigen Befestigungsmittel, insbesondere die Haken und Vorsprünge können aus an sich bekannten Materialien bestehen. Geeignet sind Nitinol, Edelstahl 316L oder Elgiloy. Auch eventuelle Baresprings können aus derartigen Materialien bestehen.

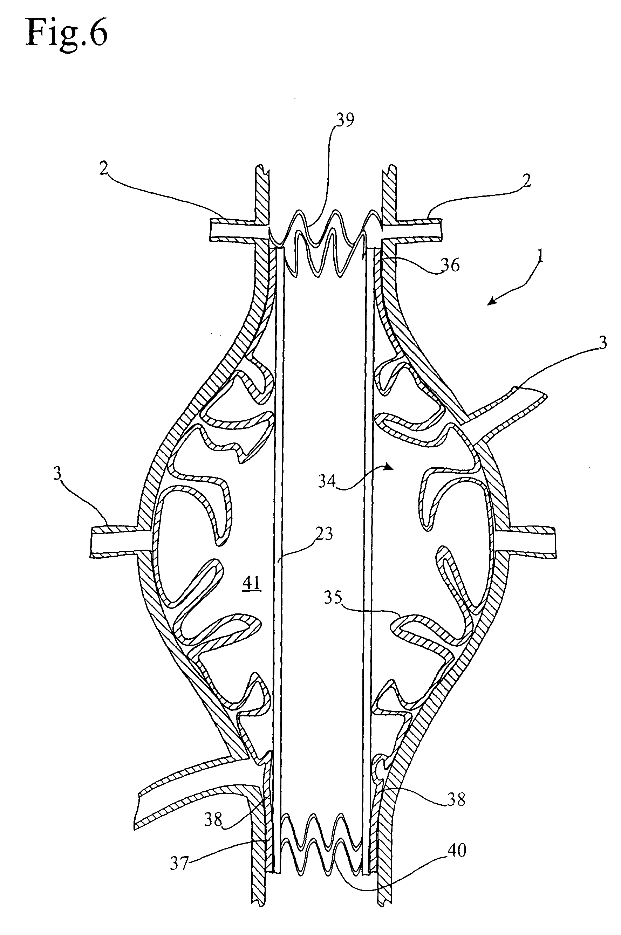

Bei der in Figur 6 dargestellten Ausführungsform ist eine Hülle 34 in gleicher Weise in ein Bauchaortenaneurysma eingesetzt wie bei den vorbeschriebenen Figuren. Ein Aorteneinsatz 23 ist in den Durchgang der Hülle zwischen proximalem Öffnungsansatz 36 und distalem Öffnungsansatz 37 eingesetzt. Seine Länge entspricht der im Aneurysma platzierten Länge der Hülle 34. Der Aorteneinsatz 23 besteht aus einem textilen Gewirk, wie es bei Gefäßprothesen üblich ist. Das Gewirk ist nicht abdichtend imprägniert, so dass Blut durch den Aorteneinsatz 23 in den Zwischenraum bzw. die Kammer 41 zwischen Aorteneinsatz 23 und Hülle 34 gelangen und diesen Zwischenraum ausfüllen kann.

Bei der Ausführungsform nach Figur 6 sind Hülle 34 und Aorteneinsatz 23 bereits abdichtend miteinander verbunden, bevor der gesamte Stentgraft implantiert wird. Es sind deshalb keine besonderen Befestigungsmittel, wie bei den vorhergehenden Ausführungsformen vorgesehen, nötig. Zur Fixierung im proximalen Bereich und im distalen Bereich reichen proximale und distale Stents 39 und 40 aus.

Figur 7 zeigt einen Katheter 42 im Querschnitt, in dem die Hülle 4 nach Figur 1 und 2 radial verjüngt angeordnet ist. In gleicher Weise kann der Aorteneinsatz 23 oder die Kombination aus Hülle 34 und Aorteneinsatz 23 im Katheter angeordnet sein, da auch der Aorteneinsatz kompressibel ist. Mit diesem Katheter wird der Stentgraft intraarteriell bis zum Aneurysma geführt und dann freigesetzt.

## Patentansprüche

1. Stentgraft-Prothese mit einer toroidförmig aufweitbaren mindestens zwei Öffnungsansätze (6, 7; 16, 17; 36, 37) aufweisenden Hülle (4; 14; 34) aus flexiblem Material zur inneren Auskleidung eines arteriellen Aneurysmas (1) und einem der Hülle (4; 14; 34) zugeordneten Aorteneinsatz (23) in Form einer Gefäßprothese mit aufweitbaren Stützstrukturen (39, 40) zur Befestigung des Aorteneinsatzes (23) in der Hülle (34) im Bereich deren Öffnungsansätze (36, 37), wobei die Hülle (4; 14; 34) als faltbares Toroid ausgebildet ist und die Form der Hülle im entfalteten Zustand der Form eines Aneurysmas angepasst ist, **dadurch gekennzeichnet, dass** die Hülle (4; 14; 34) zumindest im Mittelbereich des Toroids aus einem im wesentlichen nicht elastisch dehnbaren Material gebildet ist.

2. Stentgraft-Prothese nach Anspruch 1, **dadurch gekennzeichnet, dass** die Hülle (4; 14; 34) eine Volumenausdehnbarkeit besitzt, die mindestens dem Volumen des Aneurysmas (1) entspricht, wobei die Hülle (4; 14; 34) im Vergleich zur Größe des Aneurysmas (1) überdimensioniert ist.

3. Stentgraft-Prothese nach Anspruch 1 oder 2, **dadurch gekennzeichnet, dass** die Hülle eine bis ca. 5-fache, insbesondere 1,1-bis 2-fache der Größe des Aneurysmas aufweist.

4. Stentgraft-Prothese nach einem der vorhergehenden Ansprüche, **dadurch gekennzeichnet, dass** die Hülle (4; 14; 34) zumindest am Übergang in die Öffnungsansätze (6, 7; 16, 17; 36, 37) aus elastischem Material gebildet wird.

5. Stentgraft-Prothese nach einem der vorhergehenden Ansprüche, **dadurch gekennzeichnet, dass** die Hülle auf ihren Außenseiten mit einem Kleber zur Haftung an der Aneurysmeninnenwand versehen ist.

6. Stentgraft-Prothese nach einem der vorhergehenden Ansprüche, **dadurch gekennzeichnet, dass** die Innenseite der Hülle und/oder die Außenseite des Aorteneinsatzes die Blutkoagulation fördernde Mittel aufweisen.

7. Stentgraft-Prothese nach einem der vorhergehenden Ansprüche, **dadurch gekennzeichnet, dass** die Hülle (4; 14; 34) mindestens zum Zeitpunkt der Implantation vorzugsweise dauernd flüssigkeitsdicht ist.

8. Stentgraft-Prothese nach einem der vorhergehenden Ansprüche, **dadurch gekennzeichnet, dass** die Hülle (4; 14; 34) aus nicht resorbierbarem Material besteht.

9. Stentgraft-Prothese nach einem der Ansprüche 1 bis 8, **dadurch gekennzeichnet, dass** die Hülle (14) und der ihr zugeordnete Aorteneinsatz (23) separat vorliegen.

10. Stentgraft-Prothese nach einem der Ansprüche 1 bis 8, **dadurch gekennzeichnet, dass** der Aorteneinsatz (23) an seinen freien Enden mit den Öffnungsansätzen (16, 17) der Hülle abdichtend verbindbar ist.

11. Stentgraft-Prothese nach einem der vorhergehenden Ansprüche, **dadurch gekennzeichnet, dass** der Aorteneinsatz eine Bifurkation aufweist und die Hülle in entsprechender Weise drei Öffnungsansätze besitzt.

12. Stentgraft-Prothese nach einem der Ansprüche 1 bis 8 und 11, **dadurch gekennzeichnet, dass** der Aorteneinsatz (23) an seinen freien Enden unter Bildung einer vergrößerbaren Kammer (41) zwischen Hülle und Aorteneinsatz mit den Öffnungsansätzen (36, 37) der Hülle (34) abdichtend verbunden ist.

13. Stentgraft-Prothese nach einem der Ansprüche 1 bis 9, **dadurch gekennzeichnet, dass** ein Mantel des Aorteneinsatzes (23) für den Durchtritt von Blut durchlässig ist.

14. Stentgraft-Prothese nach Anspruch 12 oder 13, **dadurch gekennzeichnet, dass** die Kammer (41) Mittel enthält, die eine Blutkoagulation begünstigen.

15. Stentgraft-Prothese nach einem der vorhergehenden Ansprüche, **dadurch gekennzeichnet, dass** an der Außenseite der Öffnungsansätze der Hülle Einrichtungen (20) zum abdichtenden Befestigen der Prothese an den Aorteninnenwandungen vorgesehen sind.

16. Stentgraft-Prothese nach einem der vorhergehenden Ansprüche, **dadurch gekennzeichnet, dass** an der Innenseite der Öffnungsansätze der Hülle Einrichtungen (22) zum abdichtenden Befestigen des Aorteneinsatzes an der Hülleninnenwand vorgesehen sind.

17. Stengraft-Prothese nach einem der Ansprüche 1 bis 9, **dadurch gekennzeichnet, dass** der Aorteneinsatz (23) über Klettverschlüsse (22) mit der Innenseite der Öffnungsansätze der Hülle verbindbar, insbesondere verbunden ist.

## Claims

1. A stent-graft prosthesis with a toroidally expandable sleeve (4; 14; 34) having at least two opening attachments (6, 7; 16, 17; 36, 37) and made of flexible material for internal lining of an arterial aneurysm (1) and with an aortic insert (23) associated with the sleeve (4; 14; 34), in the form of a vascular prosthesis with expandable supporting structures (39, 40) for fixing the aortic insert (23) in the sleeve (34) in the region of the opening attachments (36, 37) thereof, wherein the sleeve (4; 14; 34) is designed as foldable toroid, and the shape of the sleeve in the unfolded state is adapted to the shape of an aneurysm,
**characterized in that**
the sleeve (4; 14; 34) is formed at least in the central region of the toroid of a substantially not elastically extensible material.

2. The stent-graft prosthesis according to claim 1, **characterized in that** the sleeve (4; 14; 34) has a volume extensibility which corresponds at least to the volume of the aneurysm (1), with the sleeve (4; 14; 34) being overdimensioned as compared to the size of the aneurysm (1).

3. The stent-graft prosthesis according to claim 1 or 2, **characterized in that** the sleeve has a size which is up to 5 times, in particular 1.1 to 2 times, the size of the aneurysm.

4. The stent-graft prosthesis according to any one of the preceding claims, **characterized in that** the sleeve (4; 14; 34) at least at the transition to the opening attachments (6, 7; 16, 17; 36, 37) is formed from elastic material.

5. The stent-graft prosthesis according to any one of the preceding claims, **characterized in that** the sleeve is provided on its outsides with an adhesive for bonding to the inner wall of the aneurysm.

6. The stent-graft prosthesis according to any one of the preceding claims, **characterized in that** the inside of the sleeve and/or the outside of the aortic insert include agents which promote blood coagulation.

7. The stent-graft prosthesis according to any one of the preceding claims, **characterized in that** the sleeve (4; 14; 34) is fluid-tight at least at the time of implantation, preferably permanently.

8. The stent-graft prosthesis according to any one of the preceding claims, **characterized in that** the sleeve (4; 14; 34) is made of non-absorbable material.

9. The stent-graft prosthesis according to any one of the claims 1 to 8, **characterized in that** the sleeve (14) and the aortic insert (23) associated therewith are present separately.

10. The stent-graft prosthesis according to any one of the claims 1 to 8, **characterized in that** the aortic insert (23) can be sealingly connected at its free ends to the opening attachments (16, 17) of the sleeve.

11. The stent-graft prosthesis according to any one of the preceding claims, **characterized in that** the aortic insert has a bifurcation, and the sleeve has three opening attachments in a corresponding manner.

12. The stent-graft prosthesis according to any one of the claims 1 to 8 and 11, **characterized in that** the aortic insert (23) is sealingly connected at its free ends to the opening attachments (36, 37) of the sleeve (34) to form an enlargeable chamber (41) between sleeve and aortic insert.

13. The stent-graft prosthesis according to any one of the claims 1 to 9, **characterized in that** a covering of the aortic insert (23) is pervious for blood to pass through.

14. The stent-graft prosthesis according to claim 12 or 13, **characterized in that** the chamber (41) includes means which favor blood coagulation.

15. The stent-graft prosthesis according to any one of the preceding claims, **characterized in that** devices (20) for sealing fixing of the prosthesis to the inner walls of the aorta are provided on the outside of the opening attachments of the sleeve.

16. The stent-graft prosthesis according to any one of the preceding claims, **characterized in that** devices (22) for sealing fixing of the aortic insert to the inner wall of the sleeve are provided on the inside of the opening attachments of the sleeve.

17. The stent-graft prosthesis according to any one of the claims 1 to 9, **characterized in that** the aortic insert (23) is connectable, in particular is connected, via touch and close fasteners (22) to the inside of the opening attachments of the sleeve.

## Revendications

1. Greffe d'endoprothèse comprenant une gaine (4 ; 14 ; 34) en matériau flexible pouvant être élargie sous forme toroïdale et présentant au moins deux débuts d'ouverture (6, 7 ; 16, 17 ; 36, 37) pour l'habillage interne d'un anévrisme artériel (1) et un insert aortique (23) associé à la gaine (4 ; 14 ; 34) sous forme de prothèse vasculaire ayant des structures de support pouvant être élargies (39, 40) pour la fixation de l'insert aortique (23) dans la gaine (34) dans la région de ses débuts d'ouverture (36, 37), la gaine (4 ; 14 ; 34) étant réalisée sous forme de tore pliable et la forme de la gaine, dans l'état déplié, étant adaptée à la forme d'un anévrisme, **caractérisée en ce que** la gaine (4 ; 14 ; 34), au moins dans la région centrale du tore, est formée d'un matériau sensiblement non extensible élastiquement.

2. Greffe d'endoprothèse selon la revendication 1, **caractérisée en ce que** la gaine (4 ; 14 ; 34) possède une extensibilité volumique qui correspond au moins au volume de l'anévrisme (1), la gaine (4 ; 14 ; 34) étant surdimensionnée par comparaison à la taille de l'anévrisme (1).

3. Greffe d'endoprothèse selon la revendication 1 ou 2, **caractérisée en ce que** la gaine présente jusqu'à environ 5 fois, en particulier 1,1-2 fois, la taille de l'anévrisme.

4. Greffe d'endoprothèse selon l'une quelconque des revendications précédentes, **caractérisée en ce que** la gaine (4 ; 14 ; 34), au moins au niveau de la transition aux débuts d'ouverture (6, 7 ; 16, 17 ; 36, 37), est formée d'un matériau élastique.

5. Greffe d'endoprothèse selon l'une quelconque des revendications précédentes, **caractérisée en ce que** la gaine, sur ses côtés externes, est pourvue d'une colle pour l'adhésion contre la paroi interne de l'anévrisme.

6. Greffe d'endoprothèse selon l'une quelconque des revendications précédentes, **caractérisée en ce que** le côté interne de la gaine et/ou le côté externe de l'insert aortique présentent des moyens favorisant la coagulation sanguine.

7. Greffe d'endoprothèse selon l'une quelconque des revendications précédentes, **caractérisée en ce que** la gaine (4 ; 14 ; 34), au moins à l'instant de l'implantation, est de préférence étanche de manière durable aux liquides.

8. Greffe d'endoprothèse selon l'une quelconque des revendications précédentes, **caractérisée en ce que** la gaine (4 ; 14 ; 34) se compose d'un matériau non résorbable.

9. Greffe d'endoprothèse selon l'une quelconque des revendications 1 à 8, **caractérisée en ce que** la gaine (14) et l'insert aortique (23) qui lui est associé se présentent séparément.

10. Greffe d'endoprothèse selon l'une quelconque des revendications 1 à 8, **caractérisée en ce que** l'insert aortique (23) peut être connecté de manière hermétique au niveau de ses extrémités libres aux débuts d'ouverture (16, 17) de la gaine.

11. Greffe d'endoprothèse selon l'une quelconque des revendications précédentes, **caractérisée en ce que** l'insert aortique présente une bifurcation et la gaine possède, de manière correspondante, trois débuts d'ouverture.

12. Greffe d'endoprothèse selon l'une quelconque des revendications 1 à 8 et 11, **caractérisée en ce que** l'insert aortique (23), au niveau de ses extrémités libres, est connecté de manière hermétique aux débuts d'ouverture (36, 37) de la gaine (34), en formant une chambre (41) pouvant être agrandie entre la gaine et l'insert aortique.

13. Greffe d'endoprothèse selon l'une quelconque des revendications 1 à 9, **caractérisée en ce qu'**une enveloppe de l'insert aortique (23) est perméable au passage de sang.

14. Greffe d'endoprothèse selon la revendication 12 ou 13, **caractérisée en ce que** la chambre (41) contient des moyens favorisant la coagulation sanguine.

15. Greffe d'endoprothèse selon l'une quelconque des revendications précédentes, **caractérisée en ce que** des dispositifs (20) pour la fixation hermétique de la greffe aux parois internes aortiques sont prévus au niveau du côté externe des débuts d'ouverture de la gaine.

16. Greffe d'endoprothèse selon l'une quelconque des revendications précédentes, **caractérisée en ce que** des dispositifs (22) pour la fixation hermétique de l'insert aortique à la paroi interne de la gaine sont prévus au niveau du côté interne des débuts d'ouverture de la gaine.

17. Greffe d'endoprothèse selon l'une quelconque des revendications 1 à 9, **caractérisée en ce que** l'insert aortique (23) peut être connecté, en particulier est connecté, au côté interne des débuts d'ouverture de la gaine par le biais de fermetures à bandes agrippantes (22).
